# EUROPEAN PATENT SPECIFICATION

(11) **EP 3 424 487 B1**
(45) Date of publication and mention of the grant of the patent: **15.09.2021**
(21) Application number: 17760354.5
(22) Date of filing: 03.03.2017
(51) Int. Cl.: A61K 8/73, A61Q 5/12, A61Q 5/00, A61Q 19/00, D01F 2/00, A61K 8/02, A61Q 3/00

(54) **NETWORKED DISPERSION OF BIOCELLULOSE MICROFIBRILS IN WATER**
VERNETZTE DISPERSION VON BIOCELLULOSE-MIKROFIBRILLEN N WASSER
DISPERSION EN RÉSEAU DE MICROFIBRILLES DE BIO-CELLULOSE DANS L'EAU

(30) Priority: 03.03.2016 KR 20160025765; 08.08.2016 KR 20160100599; 08.08.2016 KR 20160100603; 06.01.2017 KR 20170002301
(43) Date of publication of application: 09.01.2019
(73) Proprietor: LG Household & Health Care Ltd., Seoul 03184 (KR)
(72) Inventor: JUN, Seung-Hyun, Daejeon 34114 (KR); LEE, Seol-Hoon, Daejeon 34114 (KR); KANG, Nae-Gyu, Daejeon 34114 (KR); HUR, Eu-Gene, Daejeon 34114 (KR); KIM, Seo-Yeon, Daejeon 34114 (KR)
(74) Representative: Goddar, Heinz J.
(86) International application number: PCT/KR2017/002358
(87) International publication number: WO 2017/150950

(56) References cited:
- EP-A1- 2 526 922
- EP-A1- 2 929 874
- WO-A1-2013/094077
- JP-A- 2008 069 112
- JP-A- 2010 052 974
- KR-A- 20120 123 371
- KR-A- 20140 113 728
- US-A1- 2013 244 977
- US-A1- 2015 216 784
- US-A1- 2015 216 784

## Description

### TECHNICAL FIELD

The present disclosure relates to a water dispersion of cellulose, a method for preparing the same, and a composition including the same.

The present application claims priority to Korean Patent Application No. 10-2016-0025765 filed on March 3, 2016, Korean Patent Application No. 10-2017-0002301 filed on January 6, 2017, Korean Patent Application No. 10-2017-0027863 filed on March 3, 2017, Korean Patent Application No. 10-2016-0100599 filed on August 8, 2016, Korean Patent Application No. 10-2017-0027902 filed on March 3, 2017, Korean Patent Application No. 10-2016-0100603 filed on August 8, 2016 and Korean Patent Application No. 10-2017-0027721 filed on March 3, 2017 in the Republic of Korea.

### BACKGROUND ART

The skin functions to interrupt harmful materials from the exterior environment and to prevent moisture loss. Polymers, including natural polymers, synthetic polymers and organic polymers, applied to the skin serve to protect the skin from the exterior, to transfer active ingredients to the skin, and to prevent moisture loss as a barrier. Among such polymers, natural polymers, such as cellulose, chitosan and polysaccharides, have excellent biocompatibility and biodegradability and provide moisturizing, elasticizing and skin protecting effects.

Celluloses used currently in the human body include natural cellulose, cellulose derivatives, biocellulose, or the like. Among them, natural (naturally occurring) cellulose is present in the form of powder having a size of several tens of micrometers, is not dissolved in water and is used largely for a scrubbing agent or peeling gel. The cellulose derivatives can be obtained by substitution of hydroxyalcohol groups of cellulose and include hydroxyethyl cellulose and carboxymethyl cellulose, which are substituted so as to be dissolved in water since cellulose itself is not dissolved in water. Such water soluble celluloses are in the form of a polymer and are used largely as a thickener. Biocellulose is cellulose microfibrils prepared from bacterial cellulose and has a smaller thickness, higher crystallinity and higher physical strength as compared to plant-derived cellulose. However, since biocellulose is synthesized in the form of a sheet or gel, it is used largely for a mask pack sheet and has a difficulty in applying a cosmetic formulation.

There have been various attempts to apply a cellulose-based material to the human body. For example, Korean Patent Laid-Open No. 10-2012-0123371 (Patent Document 1) and Japanese Patent Laid-Open No. 2010-037199 (Patent Document 2) disclose cellulose fibers having a fiber diameter of at most 1000 nm or less and a number average fiber diameter of 2-150 nm, wherein the cellulose has a crystal structure of cellulose I type, and the hydroxyl group at C6 in the glucose unit of the cellulose molecule is oxidized selectively and modified into an aldehyde group and carboxyl group. It is said that the cellulose may be used for cosmetics, thickeners/gelling agents and spray compositions. However, although the cellulose disclosed in Patent Document 1 and Patent Document 2 may be used as a thickener/gelling agent to improve the shape-retaining property, dispersion stability and salt resistance, it is not suitable to realize the advantages based on cellulose on the skin. In Patent Document 1, it is stated that non-wood cellulose, such as conifer-based pulp, broad-leaved tree based pulp, cotton-based pulp, such as cotton linter or cotton lint, wheat chaff pulp and bagasse pulp ([0082]), and pulp is used in Examples. In Patent Document 2, conifer pulp is used in Examples. Additionally, EP2929874 discloses aqueous dispersions of bacterial biocellulose micro-fibrils having an average fibre diameter of 1-150 nm as a cosmetic additive. However, said document does not disclose that the alcohol groups of the biocellulose are substituted with carboxyl groups. In order to use cellulose for a cosmetic composition, it is required for cellulose to maintain its length in a micrometer scale and to retain a network among fibers even after dispersing cellulose in water. To perform micronization of such non-wood cellulose or wood-based cellulose, a pretreatment step of a top-down mode is essentially required and the cellulose length is cut randomly during the step. Thus, when the finished cellulose fibrils are introduced to a cosmetic, they may infiltrate into the skin undesirably. In addition, since it is difficult to form a network, such cellulose may be used as a thickener or formulation stabilizer but has a limitation in applying to a cosmetic composition.

Meanwhile, microdust is classified into microdust (PM10) having a diameter of 10 µm or less and ultramicro-dust (PM2.5) having a diameter of 2.5 µm or less. Microdust includes ionic ingredients, such as nitrate, ammonium salt and sulfate, carbon compounds, metallic compounds, or the like. When a man is exposed to microdust in air repeatedly for a long time, severe diseases, such as heart attack, asthma, bronchitis, pneumonia or lung cancer, may be generated. In addition, exposure of the skin to microdust results in severe problems, such as skin dryness, atopic dermatitis and dermatitis. Particularly, it has been found that microdust damages the barrier function of epidermis, worsens atopic dermatitis and is directly related with skin aging that includes increased wrinkling and pigmented spots.

Therefore, various attempts have been made to protect the skin from microdust. The most frequently used method for removing microdust is removing microdust through cleansing. Although microdust may be removed to a certain degree through cleansing, finer microdust shows higher adsorption force. Thus, it is not possible to remove microdust deposited deeply into pores completely through typical cleansing.

As a result, it is required to provide a cosmetic composition which interrupts microdust before it is adsorbed directly on the skin and prevents microdust from being adsorbed on the skin.

Meanwhile, the body hair (including hair), nail and toenail and skin include a structural protein called keratin, and they are also referred to as keratin structures. Conventionally, for the purpose of protection of such keratin, a method of covering the surface with a material, such as silicone, or a method of coating the surface by using a volatile organic solvent in the same manner as manicure has been used frequently. However, such methods are provided while not considering the characteristics of the cuticle layer formed on the outermost surface of a keratin structure like scales. Since the cuticle is very soft, the material used for protecting keratin have frequently damaged the cuticle layer when it is separated from the keratin surface. Therefore, it is required to provide a keratin structure care composition which protects keratin on the surface of a keratin structure while not adversely affecting the cuticle layer.

### DISCLOSURE

### Technical Problem

The present disclosure is directed to providing a dispersion of cellulose microfibrils which has a length and diameter of cellulose microfibrils having no possibility of infiltrating into the skin, can retain a network structure even when formulated into a water dispersed composition, and has excellent water absorbability and elasticity-enhancing ability.

The present disclosure is also directed to providing a dispersion of cellulose microfibrils which has a length and diameter of cellulose microfibrils having no possibility of infiltrating into the skin, can retain a network structure even when formulated into a water dispersed composition, and shows an excellent effect of interrupting not only microdust but also ultramicro-dust.

The present disclosure is also directed to providing a networked dispersion of biocellulose microfibrils in water which is applied to a keratin structure to protect the keratin while not adversely affecting the cuticle layer, and a method for preparing the same.

Further, the present disclosure is directed to providing a method for absorbing water and enhancing elasticity by applying the dispersion of cellulose microfibrils to the human body, a method for interrupting microdust, or a method for protecting a keratin structure.

### Technical Solution

In one aspect of the present disclosure, there is provided a networked dispersion of biocellulose microfibrils in water, wherein the alcohol groups of biocellulose are substituted with carboxyl groups, each individual microfibril in the dispersion preferably has a maximum diameter of 60-100 nm and a number average diameter of 30-60 nm, the length of biocellulose used as a raw material is maintained, and the biocellulose microfibrils are dispersed in water while forming a network, a method for preparing the same, a composition thereof, and a method for applying the dispersion and composition to the human body.

According to the present disclosure, biocellulose is selected as a cellulose material, the biocellulose is oxidized to substitute the alcohol groups of biocellulose with carboxyl groups, and the diameter and length of biocellulose are limited to obtain a networked dispersion of biocellulose microfibrils. It has been found that the resultant networked dispersion of biocellulose microfibrils maintains a length and network structure similar to those of biocellulose before oxidation, is dispersed in an aqueous phase, and has characteristics favorable to the human body, including skin setting property, high water holding ability, elasticity-enhancing ability, effect of reducing skin frictional force and thickening ability. Then, the present disclosure is finished on the basis of the use of the networked dispersion of biocellulose microfibrils for the application to the human body, such as utility thereof as a cosmetic agent or sanitary aid.

In addition, according to the present disclosure, biocellulose is selected as a cellulose material, the biocellulose is oxidized to substitute the alcohol groups of biocellulose with carboxyl groups, and the diameter and length of biocellulose are limited to obtain a networked dispersion of biocellulose microfibrils. It has been found that the resultant networked dispersion of biocellulose microfibrils retains a length and dense network structure similar to those of biocellulose before oxidation, is dispersed in an aqueous phase, does not filtrate into the skin, while providing an effect of preventing infiltration of microdust and ultramicro-dust, and can be formulated into various forms of composition through the dispersion into an aqueous phase. The present disclosure is based on this finding.

According to the present disclosure, it has been found that when biocellulose is selected as a cellulose material, the biocellulose is oxidized to substitute the alcohol groups of biocellulose with carboxyl groups, and the diameter and length of biocellulose are limited, it is possible to retain a length and network structure similar to those of biocellulose before oxidation in an aqueous phase, to provide an effect of protecting a keratin structure while not infiltrating into the skin, to prevent the cuticle layer from being damaged during removal, and to carry out formulation into various forms of compositions through the dispersion into an aqueous phase. The present disclosure is based on this finding.

The networked dispersion of biocellulose microfibrils in water, and the composition including the networked dispersion of biocellulose microfibrils in water according to the present disclosure have the following characteristics:
The networked dispersion and composition form a network in an aqueous phase. Herein, 'network' means a three-dimensional net-shaped structure of cellulose microfibrils. The term, 'aqueous phase', means a phase containing water in a solvent and covers water containing the other solvent, as long as water is contained in the solvent. For example, it includes an aqueous solution.

The networked dispersion and composition show excellent skin setting property. Herein, 'skin setting property' means that the networked dispersion of biocellulose microfibrils is applied onto the skin surface while maintaining the structural/physicochemical characteristics and/or water dispersibility of the mcirofibrils.

The networked dispersion and composition show high water holding ability. The term 'water holding ability' means force or ability of drawing water around the networked dispersion of biocellulose in water and/or force or ability of retaining the surrounding water. Water holding ability is in proportion with water absorption ability or moisturizing ability. Therefore, the networked dispersion and composition show high water absorption ability or moisturizing ability.

The networked dispersion and composition show an elasticity-enhancing effect. The term 'elasticity' means resistance against the external force, is also called 'restorative force' and is affected by the densification degree of a surface structure. Thus, it can be seen that the networked dispersion of biocellulose microfibrils in water has improved film-forming property and a subject to which the networked dispersion of biocellulose microfibrils in water shows enhanced, improved or higher resistance against external force.

The networked dispersion and composition show thickening property. The term 'thickening property' means an ability of increasing viscosity. Thus, it can be seen that the networked dispersion of biocellulose microfibrils in water has an ability of increasing viscosity while maintaining the structural and physicochemical characteristics of microfibrils.

The networked dispersion and composition show decreased frictional force. The term 'frictional force' means force by which the movement of an object in contact with a contact surface is prohibited. Skin frictional force is affected by skin roughness. Thus, it can be seen that the degree of prohibiting the movement of an object in contact with a contact surface to which the networked dispersion of biocellulose microfibrils in water is applied is decreased, softness is increased, improved or enhanced, and roughness is decreased.

The networked dispersion and composition have an effect of interrupting microdust. The term 'microdust' means a particulate material having a diameter of 10 µm or less and includes ultramicro-dust having a diameter of 2.5 µm. The expression 'interrupting microdust' means preventing or inhibiting infiltration of microdust into the skin so that microdust may be removed with ease.

The networked dispersion and composition have an effect of protecting a keratin structure. The term 'keratin structure' is a human organ or tissue including a structural protein called keratin, and examples thereof include nail, toenail, body hair, or the like. The expression 'protecting a keratin structure' means that a keratin structure is protected from being damaged or separated physically or chemically by any external cause, a keratin structure is protected from external adverse physical or chemical effects during the regeneration, recovering and healing of a damaged keratin structure, a keratin structure is protected during the removal of the dispersion according to the present disclosure or a composition including the same from the keratin structure, while not adversely affecting human tissues including the keratin structure, particularly the cuticle layer, or the like. The biocellulose microfibrils dispersed in water particularly forms a network on the surface of a keratin structure to protect separation of the surface keratin layer and is bound stably with the keratin surface, and thus is separated without any damage on the keratin surface upon the removal.

The above-described characteristics of the networked dispersion of biocellulose microfibrils in water are maintained or improved in a composition including the networked dispersion of biocellulose microfibrils in water.

The dispersion of biocellulose microfibrils in water according to the present disclosure uses biocellulose as a cellulose material.

The biocellulose used in the present disclosure means bacterial cellulose which synthesizes cellulose microfibrils directly from the cultivation of bacteria. It is differentiated from craft paper derived from various types of wood materials or sulfite pulp, powder cellulose obtained by pulverizing the same with a homogenizer or mill, or microcrystalline cellulose powder obtained by purifying the same through chemical treatment, such as acidic hydrolysis, in addition to cellulose derived from plants, such as kenaf, hemp, rice, bagasse and bamboo. Particular examples of bacteria used herein include Acetobacter, Rhizibium and Agrobacterium. When such bacteria is cultured in a medium containing a nutrition source for cultivating bacteria, bacterial cellulose is formed on the interface of the culture solution. The cultivation methods include static cultivation and agitated cultivation. The static cultivation is a method including transplanting bacteria to a medium first, and then allowing the medium in a flask to stand on a shelf, or the like, for about 10 days to carry out cultivation. Meanwhile, the agitated cultivation is a method including carrying out cultivation while performing agitation continuously at a predetermined rate in a shaking incubator through a liquid medium. Commercially available biocellulose may be used without using the cultivation of bacteria. The biocellulose has a three-dimensional network, a high crystallinity (84-89%) and sufficient pores. The biocellulose has a length of several micrometers to several tens of micrometers and has a diameter with predetermined, i.e., uniform length distribution.

In the networked dispersion of biocellulose microfibrils in water according to the present disclosure, the alcohol groups in the biocellulose according to the present disclosure are substituted with carboxyl groups.

A least 0.8 mmol/g or more, preferably 1.0 mmol/g or more of cellulose of the total alcohol groups contained in the biocellulose are substituted with carboxyl groups.

Substitution of carboxyl groups in the biocellulose with carboxyl groups may be carried out by using various methods known in the art. For example, the substitution may be carried out by adding an oxidizing agent and biocellulose together to distilled water containing an N-oxyl compound dissolved therein, followed by agitation.

In the networked dispersion of biocellulose microfibrils in water according to the present disclosure, the alcohol groups of the biocellulose are substituted with carboxyl groups. The networked dispersion shows a negative charge of -60 mV to -90 mV.

The negative charge of the networked dispersion of biocellulose microfibrils in water according to the present disclosure causes repulsion to the negative charge of microdust, thereby reducing attachment of microdust.

Each individual microfibril in the networked dispersion of biocellulose microfibrils in water according to the present disclosure has a number average diameter of 30-60 nm. Within the above-defined range, the networked dispersion is applied advisably to the human body. Particularly, the networked dispersion is favorable to use as a cosmetic composition and sanitary aid composition. When the number average diameter is 30-60 nm, it is possible to realize a unique function of preventing scrubbing during application while forming a network structure and to interrupt microdust effectively while not causing infiltration to the skin. Within the above-defined range, the networked dispersion is applied advisably to the human body. For example, it is applied advisably to a cosmetic composition and a sanitary aid composition.

In addition, each individual microfibril in the networked dispersion of biocellulose microfibrils in water according to the present disclosure has a length similar to the length of the biocellulose used as a raw material. In other words, the microfibrils according to the present disclosure maintain the length of the biocellulose used as a raw material. The expression '... has a length similar to the length ...' or 'maintains the length' means that there is substantially no difference between the length of each microfibril and that of the biocellulose used as a raw material. The expression 'is substantially no difference' means that the difference in length is ± 10% or less, ± 5% or less, ± 1% or less, preferably ± 0.01% or less. In the process for preparing the dispersion according to the present disclosure, a step of cutting microfibrils is not included, and thus the length of the biocellulose as a raw material is maintained substantially.

The networked dispersion of biocellulose microfibrils in water according to the present disclosure maintains structural and physicochemical characteristics as microfibrils and/or water dispersibility, when it is added to an aqueous solution. Particularly, the networked dispersion is dispersed stably while maintaining its unique network.

The networked dispersion of biocellulose microfibrils in water according to the present disclosure may be formulated into a water dispersible composition.

The term 'water dispersible composition' means a composition including water as a solvent, i.e., a composition including a dispersion of biocellulose microfibrils in water according to the present disclosure is dispersed in an aqueous phase. The composition may be an aqueous phase alone, or may be a water-in-oil, oil-in-water or multi-emulsion, but is not limited thereto.

The networked dispersion of biocellulose microfibrils in water according to the present disclosure may be prepared by using various methods known to those skilled in the art with no particular limitation. However, the networked dispersion uses biocellulose as a raw material, and thus requires no pretreatment step in a top-down mode. For example, there is no need for a step of cutting microfibrils.

Preferably, the networked dispersion according to the present disclosure may be obtained by the method including the following steps of:
dissolving an N-oxyl compound into water;
adding biocellulose and an oxidizing agent to the resultant solution, followed by agitation; and
removing the reactants through washing.

The N-oxyl compound that may be used according to the present disclosure is a catalyzing agent and is used to accelerate oxidation as disclosed herein. For example, the N-oxyl compound may be at least one selected from 2,2,6,6-tetramethyl-1-piperidine-oxy radical (TEMPO); 4-hydroxy TEMPO derivative having suitable hydrophobicity imparted by etherifying the hydroxyl group with an alcohol or esterifying the hydroxyl group of 4-hydroxy-2,2,6,6-tetramethyl-1-piperidine-oxy radical (4-hydroxy TEMPO) with a carboxylic acid or sulfonic acid; and aza-adamantane type nitroxy radical. Most preferably, 2,2, 6,6-tetramethyl-1-piperidine-oxy radical (TEMPO) may be used. The N-oxyl compound may be used in any amount with no particular limitation, as long as it is used in such an amount that it can form microfibrils of biocellulose. For example, the N-oxyl compound may be used in an amount of 0.01-100 mmol, preferably 0.01-50 mmol, more preferably 0.05-30 mmol based on 1 g of a dried cellulose-based raw material.

The oxidizing agent that may be used herein preferably includes halogen, hypohalogenous acid, halogenous acid, perhalogenous acid, or salts thereof, halogen oxide, halogen peroxide, or the like. Among halogens, chlorine is used preferably. For example, chlorine, hypochlorite, chlorite, hyperchloric acid or salts thereof, chlorine oxide, or chlorine peroxide is preferred. Most preferably, sodium hypochlorite is used. Sodium hypochlorite is cost-efficient in terms of the cost required for producing microfibrils. It is particularly preferred, since it is most widely used in the current industrial processes, is cheap and causes low environmental load. The oxydizing agent is used in such an amount that it can accelerate oxidation. For example, the oxidizing agent may be used in an amount of 0.5-500 mmol, preferably 0.5-50 mmol, more preferably 2.5-25 mmol based on 1 g of a dried cellulose-based raw material.

In the method for preparing a networked dispersion of microfibrils in water according to the present disclosure, bromide is not used substantially.

The expression 'is not used substantially' means that bromide is used in an amount of 0.1 mmol or less, preferably 0.01 mmol or less, more preferably 0.001 mmol or less based on 1 g of a dried cellulose-based raw material. Bromide, such as chlorine bromide may have toxicity problems in the bronchus or lung and safety problems during its handling and may cause environmental pollution due to heavy metals upon disposal. Thus, such bromide is not used substantially according to the present disclosure. According to the present disclosure, it is possible to prepare a networked dispersion of biocellulose microfibrils in water having excellent efficacy without using bromide.

In the method for preparing a networked dispersion of biocellulose microfibrils in water according to the present disclosure, oxidation may be carried out smoothly even under a mild condition of room temperature of about 15-30°C.

Since carboxyl groups are produced in the biocellulose structure as oxidation proceeds, pH of the reaction mixture is decreased. Therefore, for the purpose of efficient oxidation, it is required to maintain pH of the reaction mixture at 8-12, preferably about 10-11 by adding an alkaline solution continuously thereto. The reaction time of oxidation may be set suitably and is not limited particularly, but may be about 0.5- 60 hours, preferably about 1-50 hours, more preferably about 24-48 hours.

The networked dispersion of biocellulose microfibrils in water according to the present disclosure may be used for various types of compositions, particularly for a cosmetic composition and sanitary aid composition. In addition, the networked dispersion of biocellulose microfibrils in water according to the present disclosure has high skin adhesion while not causing infiltration into the skin, and thus may be used for a cosmetic composition and sanitary aid composition applied to the human body, preferably to the skin.

The networked dispersion of biocellulose microfibrils in water according to the present disclosure is dispersed characteristically while forming a network in an aqueous phase, and thus is particularly useful for a composition including an aqueous phase.

More particularly, the networked dispersion of biocellulose microfibrils in water according to the present disclosure has skin setting property, water retention ability, elasticity-enhancing property, thickening property and softness improving ability, and thus may be used for applications based on the same. The networked dispersion of biocellulose microfibrils in water according to the present disclosure or a composition including the same may be used for increasing water content or enhancing elasticity.

More particularly, the networked dispersion of biocellulose microfibrils in water according to the present disclosure has high skin adhesion while not causing infiltration into the skin, and shows an excellent effect of interrupting microdust. Thus, it can be used for some applications based on this. The networked dispersion of biocellulose microfibrils in water according to the present disclosure or a composition including the same may be used for interrupting microdust.

More particularly, the networked dispersion of biocellulose microfibrils in water according to the present disclosure has high skin adhesion while not causing infiltration into the skin, and shows an excellent effect of protecting a keratin structure. Thus, it can be used for some applications based on this. The networked dispersion of biocellulose microfibrils in water according to the present disclosure or a composition including the same may be used for protecting a keratin structure.

In another aspect of the present disclosure, there is provided a cosmetic composition including a networked dispersion of biocellulose microfibrils in water, wherein the biocellulose microfibrils have a diameter of at most 60-100 nm and a number average diameter of 30-60 nm and are dispersed while forming a network in an aqueous phase.

The cosmetic composition according to the present disclosure may be used as a cosmedical (cosmeceutical).

Herein, the term 'cosmedical (cosmeceutical)' means a cosmetic to which a special treatment function of a pharmaceutical is introduced so that it may have a special function with an emphasized physiological effect or efficacy. Particular examples of the cosmedical (cosmeceutical) include a product which helps skin whitening, product which helps improvement of skin wrinkles, product which helps tanning skin nicely or protecting the skin from UV rays, or other cosmetic products defined by the orders of Ministry of Health and Welfare.

The cosmetic composition according to the present disclosure may be used as an ingredient of a general cosmetic product or cosmedical product together with adjuvants used conventionally in the cosmetic field, such as a fat material, organic solvent, solubilizing agent, concentrating agent, gelling agent, softening agent, antioxidant, suspending agent, stabilizing agent, foaming agent, fragrance, surfactant, water, ionic or nonionic emulsifier, filler, metal ion blocker, chelating agent, preservative, vitamin, blocking agent, wetting agent, essential oil, dye, pigment, hydrophilic or oleophilic active ingredient, and lipid vesicles, as well as adjuvants used conventionally in the field of cosmetics or dermatology.

The cosmetic product including the cosmetic composition according to the present disclosure may be provided in any formulations used conventionally in the art. For example, it may be formulated into solution, suspension, emulsion, paste, gel, cream, lotion, powder, soap, surfactant-containing cleansing, oil, powder foundation, emulsion foundation, wax foundation, spray, or the like, but is not limited thereto. More particularly, it may be provided in the formulation of skin softener, astringent, nourishing skin, nourishing cream, massage cream, water cream, water gel cream, lotion, gel, essence, eye cream, peel-off mask pack, cleansing cream, cleansing foam, cleansing water, pack, ointment, stick, patch, spray or powder.

The networked dispersion of biocellulose microfibrils in water may be used in an amount of 0.0001-10, preferably 0.0001-5, more preferably 0.0005-5 dry wt% based on the total weight of the cosmetic composition.

In still another aspect of the present disclosure, there is provided a sanitary aid composition including a networked dispersion of biocellulose microfibrils in water, wherein the biocellulose microfibrils have a diameter of at most 60-100 nm and a number average diameter of 30-60 nm and are dispersed while forming a network in an aqueous phase.

As used herein, the term 'sanitary aid' means any one article selected from an article used for treating, alleviating, healing or preventing conditions harmful to the humans or animals, article which has a weak function to the human body or does not act directly to the human body, and is not an instrument or machine or one similar thereto, and an article corresponding to a formulation which is for use in sterilization, insect killing or other similar applications for the purpose of preventing infection, is used for the purpose of diagnosing, treating, alleviating, healing or preventing diseases of humans or animals, and is not an instrument, machine or apparatus, and an article which is used for the purpose of providing a pharmaceutical effect upon the structure and function of humans or animals, except for one that is not a machine or apparatus. The sanitary aid also includes a skin application agent and personal hygienic article.

As used herein, 'skin application agent' means a solid, semi-solid or liquid external application medicine prepared by mixing a medicine with various base materials, such as oil and fats, Vaseline, lanoline, glycerol, or the like in order to facilitate application onto the skin. The external application formulation is not particularly limited but particular examples thereof include powder, gel, ointment, cream, liquid or aerosol formulation.

The networked dispersion of biocellulose microfibrils in water according to the present disclosure may be present in an amount of 0.0001-10, preferably 0.0001-5, more preferably 0.0005-5 dry wt% based on the total weight of the sanitary aid composition.

The networked dispersion of biocellulose microfibrils in water according to the present disclosure or a composition including the same has skin setting property, water holding ability, elasticity enhancing property, thickening property and softness improving ability, and thus may be used advisably based on this.

More particularly, the networked dispersion of biocellulose microfibrils in water according to the present disclosure or a composition including the same may be applied to the human body so that the water content may be increased or elasticity may be enhanced.

The human body preferably includes the skin.

The term 'application' includes any application mode as long as it directly or indirectly affects the human body but may include applying onto the human body.

The networked dispersion of biocellulose microfibrils in water according to the present disclosure or a composition including the same has microdust-interrupting ability and thus may be used advisably based on this.

More particularly, the networked dispersion of biocellulose microfibrils in water according to the present disclosure or a composition including the same may be applied to the human body so that microdust may be interrupted.

The human body preferably includes the skin.

The term 'application' includes any application mode as long as it directly or indirectly affects the human body but may include applying onto the human body.

The networked dispersion of biocellulose microfibrils in water according to the present disclosure or a composition including the same may be applied to the human body so that a keratin structure may be protected.

The human body preferably includes the skin.

The term 'application' includes any application mode as long as it directly or indirectly affects the human body but may include applying onto the human body.

The networked dispersion of biocellulose microfibrils in water according to the present disclosure or a composition including the same may be used to protect various keratin structures. For example, when it is applied to the hair, it may be formulated into at least one selected from a pre-shampoo composition, shampoo, rinse, treatment, wax, gel, spray, moose, hair lotion, hair pack, hair essence, hair cream, permanent dyeing agent, temporary dyeing agent, perming agent, nonwoven web and a sheet. In addition, when it is applied to the nail or toenail, it may be formulated into at least one formulation selected from a base for trimming or protecting nail and toenail, manicure, topcoat, nourishing agent, reinforcing agent and gel.

### Advantageous Effects

The networked dispersion of biocellulose microfibrils in water according to the present disclosure maintains the length of biocellulose as a raw material, retains a fibrous network even after being formulated into a composition, provides excellent skin setting property, moisture absorbing property and elasticity-enhancing effect from the microfibrils formed on the skin, has an effect of improving softness, and thus may be used advisably as a sanitary aid and cosmetic product.

### DESCRIPTION OF DRAWINGS

The accompanying drawings illustrate a preferred embodiment of the present disclosure and together with the foregoing disclosure, serve to provide further understanding of the technical features of the present disclosure, and thus, the present disclosure is not construed as being limited to the drawing.
FIG. 1 shows (a) a photographic view of the cellulose microfibrils according to Comparative Example 1, and (b) a photographic view of the networked dispersion of cellulose microfibrils in water after chemical treatment according to Example 1.
FIG. 2 shows (a, b) photographic views of the lignum-based cellulose dispersion at a concentration of 0.05% and 01015% according to Comparative Example 2; and (c, d) photographic views of the networked dispersion of biocellulose microfibrils at a concentration of 0.05% and 0.015% according to Example 1.
FIG. 3 is a scanning electron microscopic (SEM) image of the skin of a pig before and after the application of Example 1.
FIG. 4 shows (a) a graph illustrating variations of water contact angle as a function of time in the skin of a pig to which cellulose according to Examples or Comparative Examples are applied, and (b) a microscopic image of the initial contact angle.
FIG. 5 is a graph illustrating the frictional force data measured after applying Example 1 to the skin.
FIG. 6 is a graph illustrating the viscosity data of essence formulations (Comparative Example 5, and Examples 2 and 3).
FIG. 7 is a graph illustrating variations of water contact angle as a function of time in the skin of a pig to which each of Comparative Example 5 and Example 2 is applied.
FIG. 8 shows (a) a scanning electron microscopic (SEM) image of the skin of a pig to which Comparative Example 6 is applied, and (b) SEM image of the skin of a pig to which 0.01 dry wt% of Example 1 is applied, and (c, d) SEM image of the skin of a pig to which 0.05 dry wt% of Example 1 is applied.
FIG. 9 shows (a) a scanning electron microscopic (SEM) image of the skin of a pig to which 0.05 dry wt% of Example 1 is applied and then microdust is applied thereon, and (b) SEM image of the skin of a pig to which 0.05 dry wt% of Comparative Example 6 is applied and then microdust is applied thereon.

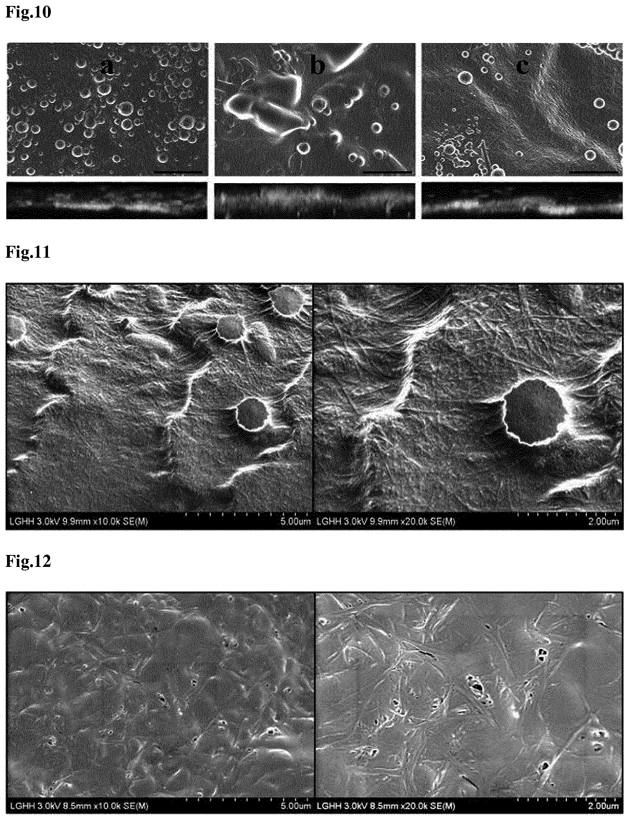

FIG. 10 shows (a) a SEM image (upper part) of the skin of a pig to which Comparative Example 6 is applied and then substitute microdust is applied thereto and fluorescence microscopic image (lower part) thereof, (b) SEM image (upper part) of the skin of a pig to which 0.05 dry wt% of Comparative Example 7 is applied and then substitute microdust is applied thereto and fluorescence microscopic image (lower part) thereof, and (c) SEM image (upper part) of the skin of a pig to which 0.05 dry wt% of Example 1 is applied and then substitute microdust is applied thereto and fluorescence microscopic image (lower part) thereof.
FIG. 11 is a photographic view of the surface of human hair taken with scanning electron microscopy (SEM), after the hair is treated with essence having a formulation of O/W emulsion containing the networked dispersion of biocellulose microfibrils in water according to Example 6, and then dried.
FIG. 12 is a photographic view of the surface of human nail taken with scanning electron microscopy (SEM), after the nail is treated with essence having a formulation of O/W emulsion containing the networked dispersion of biocellulose microfibrils in water according to Example 6, and then dried.

### BEST MODE

Hereinafter, preferred embodiments of the present disclosure will be described in detail with reference to the accompanying drawings. However, it should be understood that the embodiments may be modified into various forms and the scope of the present disclosure is not limited to the following embodiments. The following embodiments are provided to describe the present disclosure more perfectly to those skilled in the art.

### Preparation Example 1: Chemical Treatment of Biocellulose

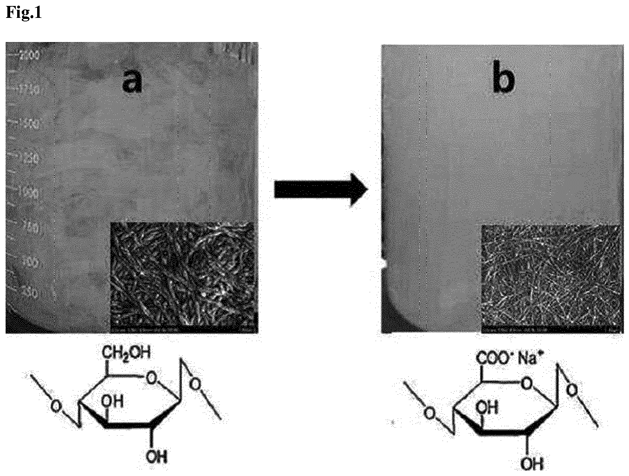

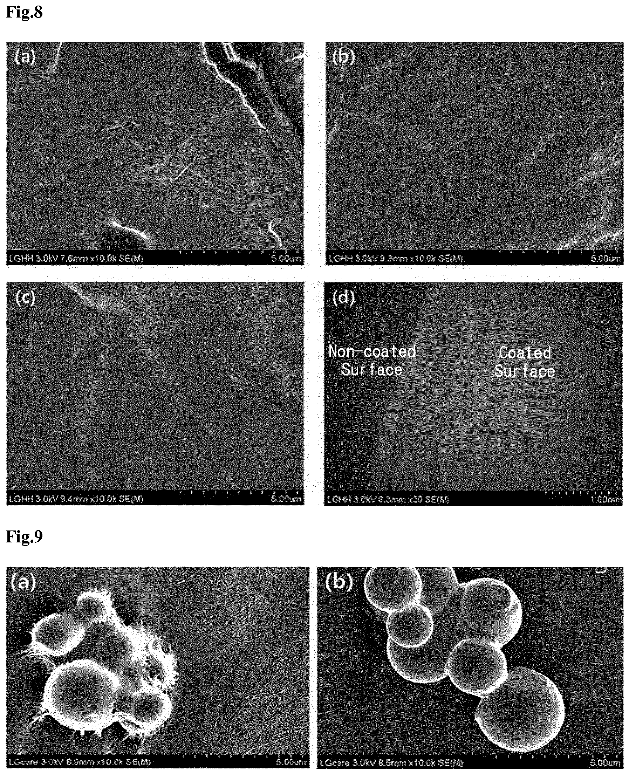

To carry out chemical treatment of biocellulose, Biocellulose (Ezcostec, non-perforated sheet), sodium hypochlorite and 2,2,6,6-tetramethyl-1-piperidine-N-oxy radical (hereinafter, referred to as TEMPO) catalyst were used. To 100 g of distilled water, 10 mg of TEMPO catalyst was dissolved, 5 g of a biocellulose sheet was introduced and 8 g of sodium hypochlorite were added. Then, the reaction materials were agitated at room temperature for 12-24 hours while maintaining pH of 10 or more to obtain microfibrils including the biocellulose sheet dispersed in water (b in FIG. 1, b in FIG. 8). The networked dispersion of biocellulose microfibrils in water was subjected to purification and washing, and then stored at room temperature. In the resultant networked dispersion of biocellulose microfibrils (Example 1), the microfibrils had a diameter of at most 60-100 nm and a number average diameter of 30-60 nm, and maintained the length of Biocellulose (Ezcostec, non-perforated sheet).

To determine whether Example 1 had a network structure of microfibrils or not, scanning electron microscopy (SEM) was used. As a result, it was determined that the biocellulose dispersed in water had a network structure of microfibrils (b in FIG. 1, b is FIG. 8, c, d in FIG. 8).

### Examples and Comparative Examples Used for Test Examples 1-7

Example 1 is a networked dispersion of biocellulose microfibrils obtained according to Preparation Example 1.

Comparative Example 1 was obtained by introducing the biocellulose sheet into distilled water, followed by agitation.

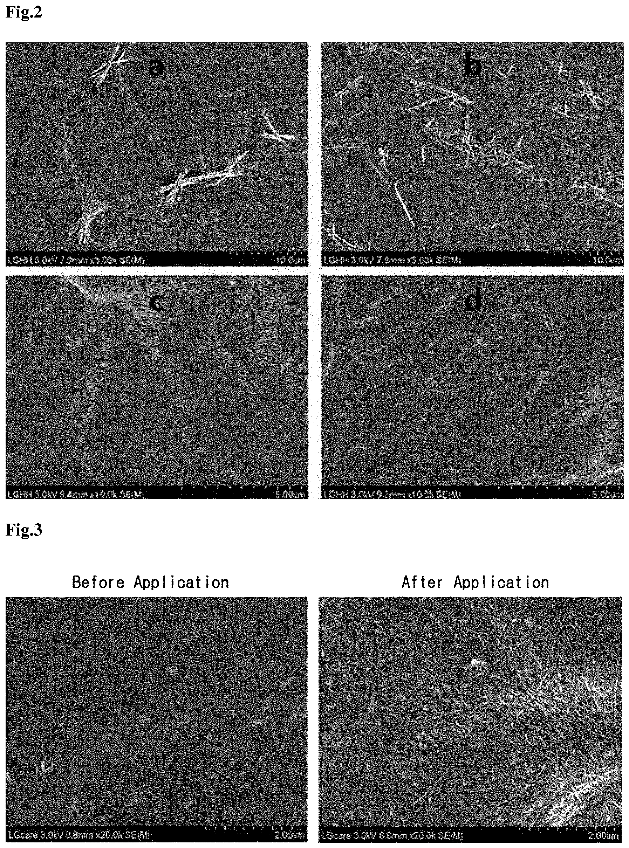

Comparative Example 2 was lignum-based cellulose prepared in the same manner as Example 1. SEM images of Comparative Example 2 are shown in a in FIG. 1 and a, b in FIG. 2.

Comparative Example 3 was prepared by introducing carboxymethyl cellulose as currently used water soluble cellulose into distilled water, followed by agitation.

Comparative Example 4 was prepared by introducing hydroxyethyl cellulose as currently used water soluble cellulose into distilled water, followed by agitation.

Comparative Example 5 is an essence formulation containing no Example 1.

Example 2 is an essence formulation containing 0.015 dry wt% of the networked dispersion of biocellulose microfibrils in water according to Example 1.

Example 3 is an essence formulation containing 0.03 dry wt% of the networked dispersion of biocellulose microfibrils in water according to Example 1.

### Test Example 1: Comparison of Networked Dispersion of Biocellulose Microfibrils in Water with Dispersion of Lignum-Based Cellulose

A test was carried out to compare the networked dispersion of biocellulose microfibrils in water with a dispersion of oxidized lignum-based cellulose. Comparative Example 2 obtained by subjecting a raw material of lignum-based cellulose (Whatman, filter paper, 1005-110) to the same process as Preparation Example 1 was determined through SEM.

FIG. 2 shows dilution of Comparative Example 2 to 0.05 dry wt% in portion a and dilution of Comparative Example 2 to 0.015 dry wt% in portion b. As can be seen from a and b in FIG. 2, Comparative Example 2 shows a needle-like structure and does not form a network.

On the contrary, FIG. 2 shows dilution of the same concentration of Example 1 to 0.05 dry wt% in portion c and dilution thereof to 0.015 dry wt% in portion d. As can be seen from c and d in FIG. 2, Example 1 has a number average diameter of 30-60 nm and maintains a length of several micrometers to several tens of micrometers, thereby forming a network among fibrils even at a low concentration. Thus, it can be seen that lignum-based cellulose is different from biocellulose in terms of the structure of network formation, and thus shows different functions.

### Test Example 2: Determination of Skin Setting Networked Dispersion of Biocellulose Microfibrils in Water

To determine whether or not Example 1 was set on the skin when 0.03 dry wt% of Example 1 was applied to the skin, it was applied to the skin (2 cm x cm) of a pig, dried thereon and observed through SEM. As can be seen from FIG. 3, it was shown that a networked dispersion of cellulose microfibrils was applied stably to the surface of the skin of a pig. This demonstrates that Example 1 forms microfibrils and is set stably on the skin surface.

### Test Example 3: Determination of Water Holding Ability of Networked Dispersion of Biocellulose Microfibrils in Water

To determine water holding ability, each of Comparative Examples 2, 3 and 4 and Example 1 was applied to the skin surface of a pig at a concentration of 0.5 dry wt% and water was dropped thereto to measure the contact angle. The results are shown in the following Table 1. The initial contact angle was as follows: Comparative Example 4 > Comparative Example 2 > Comparative Example 3 > Example 1. A smaller contact angle suggests higher hydrophilicity.

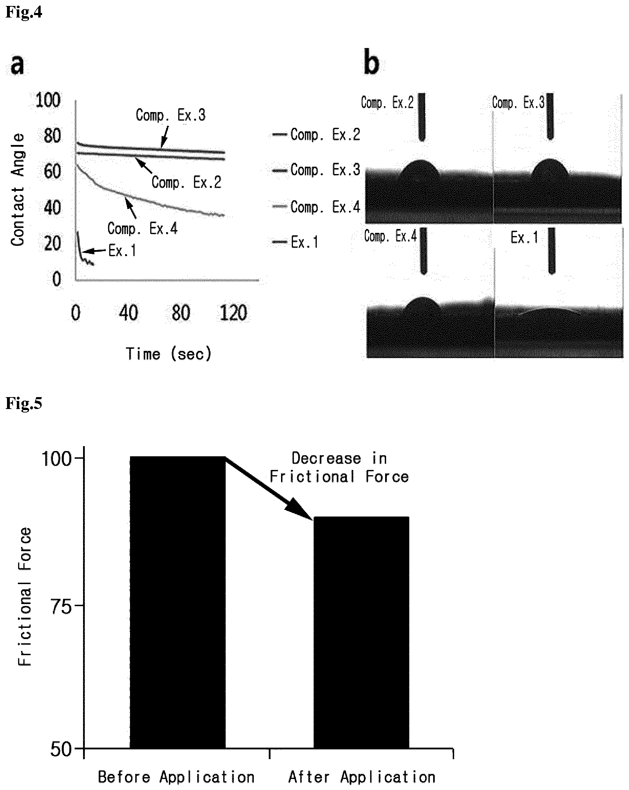

In addition, variations in contact angles are shown in FIG. 4 as a function of time. In the case of Example 1, the contact angle is decreased rapidly, which suggests that the cellulose microfibrils dispersed in water draw water rapidly. This demonstrates that the cellulose microfibrils dispersed in water shows increased water holding ability and thus provides high moisturizing ability.

**[Table 1]**

| | Comp. Ex. 2 | Comp. Ex. 3 | Comp. Ex. 4 | Ex. 1 |
|---|---|---|---|---|
| Contact Angle | 70.8 | 64.1 | 76.5 | 19.6 |

### Test Example 4: Determination of Skin Frictional Force of Networked Dispersion of Biocellulose Microfibrils in Water

First, 0.015 dry wt% of a dispersion of cellulose in water was applied to the skin. Then, 20 minutes after the application, a friction meter was used to determine skin frictional force. As a result, it was shown that skin frictional force was reduced by 10% as compared to the skin frictional force before the application (FIG. 5), and the networked dispersion of cellulose microfibrils remaining on the skin softened the skin.

### Test Example 5: Determination of Thickening Ability of Networked Dispersion of Biocellulose Microfibrils in Water

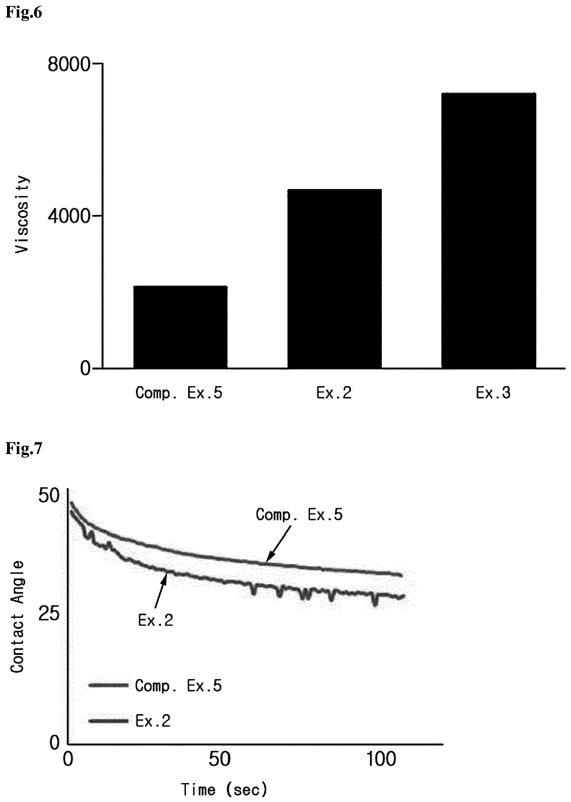

An essence formulation was prepared by using the composition as shown in the following Table 2 (unit: wt%). Each of the resultant skin cosmetic agents was determined for its viscosity. It was shown that the viscosity is increased as the proportion of Example 1 is increased (FIG. 6). This demonstrates that Example 1 shows the same thickening ability as the conventional cellulose while retaining the structure of microfibrils.

**[Table 2]**

| | Material | Comp. Ex. 5 | Ex. 2 | Ex. 3 |
|---|---|---|---|---|
| 1 | Dimethicone | 1.00 | 1.00 | 1.00 |
| 2 | Pentaerythrytyl tetraethylhexanoate | 3.00 | 3.00 | 3.00 |
| 3 | Hydrogenated polydecene | 1.00 | 1.00 | 1.00 |
| 4 | Dipropylene glycol | 7.00 | 7.00 | 7.00 |
| 5 | Glycerine | 7.00 | 7.00 | 7.00 |
| 6 | Methyl glucose sesquistearate | 0.40 | 0.40 | 0.40 |
| 7 | Cyclopentasiloxane | 3.00 | 3.00 | 3.00 |
| 8 | Purified water | Up to 100 | Up to 100 | Up to 100 |
| 9 | Example 1 | - | 0.015 | 0.03 |
| 10 | Xanthan gum | 4.00 | 4.00 | 4.00 |
| 11 | Carbomer | 12.00 | 12.00 | 12.00 |
| 12 | Preservative | 1.50 | 1.50 | 1.50 |
| 13 | Sorbitol | 1.50 | 1.50 | 1.50 |
| 14 | Chelating agent | 0.40 | 0.40 | 0.40 |
| 15 | Anti-inflammatory agent | 2.00 | 2.00 | 2.00 |
| 16 | pH modifier | 2.00 | 2.00 | 2.00 |

### Test Example 6: Determination of Water Holding Ability of Formulation Containing Networked Dispersion of Biocellulose Microfibrils in Water

To determine the water holding ability of a networked dispersion formulation of cellulose microfibrils in water, essence formulations were prepared (Comparative Example 5, Example 2) in the presence/absence of the dispersion of cellulose microfibrils (the composition (wt%) was the same as Table 2) and were applied to the skin of a pig, and then water was dropped thereto to determine the contact angle. As a result, the formulation containing Example 1 shows a larger decrease in contact angle with time (FIG. 7). It is thought this is because the cellulose microfibrils maintain the property of drawing and absorbing water even though they are included in the formulation.

### Test Example 7: Determination of Elasticity-enhancing Effect of Networked Dispersion of Biocellulose Microfibrils in Water

To determine the effects of Example 1, a test for measuring elasticity was carried out. A predetermined concentration of gelatin was melted on a well plate, followed by solidification, to provide gel. Then, a networked dispersion of cellulose microfibrils in water was applied thereto and elasticity was measured by using a texture analyzer. A sample having a size smaller than the area of the gelatin gel was mounted to the texture analyzer and was dropped at a predetermined rate so that the gelatin gel was pushed, and the pressure at that time was measured. When the elasticity was increased, the surface structure was densified. Thus, this test used the principle that the resistance force is increased when the sample is pushed by a predetermined extent of force. When the following in-vitro elasticity evaluation method was used to determine Comparative Example 2 and 0.03 dry wt% of Example 1, Example 1 showed an increase in elasticity by about 20% as compared to Comparative Example 2. It is thought that the cellulose microfibrils form a film and increases elasticity. In addition, improvement of elasticity was determined with 10 subjects. It was shown that elasticity was increased significantly.

### Examples and Comparative Examples Used for Test Examples 8-12

Example 1 is a networked dispersion of biocellulose microfibrils obtained according to Example 1.

Comparative Example 6 is distilled water.

Comparative Example 7 is xanthan gum, which is a water soluble polymer.

Comparative Example 8 is an essence formulation containing no Example 1.

Comparative Example 9 is a skin formulation containing no Example 1.

Example 4 is an essence formulation containing 0.05 dry wt% of Example 1.

Example 5 is a skin formulation containing 0.05 dry wt% of Example 1.

### Test Example 8: Determination of Skin Setting for Composition of Networked Dispersion of Biocellulose Microfibrils in Water

To determine whether Example 1 maintains the original microfibril structure or not, scanning electron microscopy was carried out. As a result, it was shown that cellulose maintained the shape of microfibrils (b in FIG. 8, c, d in FIG. 8 and a in FIG. 9).

To determine whether or not 0.01 dry wt% of Example 1 or 0.05 dry wt% of Example 1 was set stably on the skin upon skin application, Example 1 was applied to the skin (2 cm x 2 cm) of a pig, dried and observed by SEM. As can be seen from FIG. 1, the skin surface of a pig is also coated with the composition of networked dispersion of biocellulose microfibrils. This demonstrates that Example 1 is set stably on the skin surface while forming microfibrils. In addition, to determine how much the skin is coated with 0.05 dry wt% of Example 1, the interface between the coated portion and the non-coated portion was observed. As a result, it was shown that all coated portion was covered with the composition of networked dispersion of biocellulose microfibrils in water. This demonstrates that the composition of networked dispersion of biocellulose microfibrils in water covers most of the skin when it is applied to the skin so that microdust may not be attached to the skin.

### Test Example 9: Determination of Charges for Composition of Networked Dispersion of Biocellulose Microfibrils in Water

To determine the charges of Example 1, the essence formulation as shown in the following Table 3 was prepared and Comparative Example 8 and Examples 1 and 4 were determined for zeta potential. The surface charge value of each of Examples 1 and 4 and Comparative Example 8 are shown in the following Table 4. In the case of Example 1, it is negatively charged with a surface charge value of -77.5 mV. In the case of Example 2, it is negatively charged with a surface charge value of -70.7 mV. But, in the case of Comparative Example 8, it is negatively charged with a surface charge value of -7.1 mV. In the determination of zeta potential, a value between -20 mV and +20 mV does not suggest a stable charge. Thus, it cannot be said that Comparative Example 8 is negatively charged. Therefore, since the carboxyl groups of the composition of networked dispersion of biocellulose microfibrils in water are negatively charged, it can be seen that stable negative charges are observed when the composition is applied to a formulation.

**[Table 3]**

| | Material | Comp. Ex. 8 | Ex. 4 |
|---|---|---|---|
| 1 | Dimethicone | 1.00 | 1.00 |
| 2 | Pentaerythrytyl tetraethylhexanoate | 3.00 | 3.00 |
| 3 | Hydrogenated polydecene | 1.00 | 1.00 |
| 4 | Dipropylene glycol | 7.00 | 7.00 |
| 5 | Glycerine | 7.00 | 7.00 |
| 6 | Methyl glucose sesquistearate | 0.40 | 0.40 |
| 7 | Cyclopentasiloxane | 3.00 | 3.00 |
| 8 | Purified water | Up to 100 | Up to 100 |
| 9 | Example 1 | - | 0.05 |
| 10 | Xanthan gum | 4.00 | 4.00 |
| 11 | Carbomer | 12.00 | 12.00 |
| 12 | Preservative | 1.50 | 1.50 |
| 13 | Sorbitol | 1.50 | 1.50 |
| 14 | Chelating agent | 0.40 | 0.40 |
| 15 | Anti-inflammatory agent | 2.00 | 2.00 |
| 16 | pH modifier | 2.00 | 2.00 |

**[Table 4]**

| | Ex. 1 | Ex. 4 | Comp. Ex. 8 |
|---|---|---|---|
| Surface charge (mV) | -77.5 | -70.7 | -7.1 |

### Test Example 10: Determination of Microdust Attachment for Composition of Networked Dispersion of Biocellulose Microfibrils in Water

After each of 0.05 dry wt% of Example 1, Comparative Example 6 and 0.05 dry wt% of Comparative Example 7 was applied to the skin of a pig, and substitute microdust with a size of 1-5 µm was applied to the samples, including the non-treated control, SEM and fluorescence microscopy were carried out to determine whether the substitute microdust was in direct contact with the skin surface or not. In the case of fluorescence microscopy, the substitute microdust was treated with red fluorescence, the skin was treated with green fluorescence, and the applied water soluble polymer and dispersion of biocellulose microfibrils in water were not treated with fluorescence, so that whether red particles were in direct contact with the green skin or not depending on materials applied to the skin.

As can be seen from FIG. 9 and FIG. 10, when the skin was treated with distilled water, it was shown that microdust was in direct contact with the skin (b in FIG. 9 and a in FIG. 10). When the water soluble polymer was applied, it was shown that microdust infiltrated into the water soluble polymer and was in direct contact with the skin (b in FIG. 10). On the contrary, in the case of the dispersion of biocellulose microfibrils in water, the dense network structure (interval between microfibrils: 10 nm or less) interrupted microdust from being in direct contact with the skin (a in FIG. 9, c in FIG. 10).

### Test Example 11: Determination of Microdust Attachment Ratio for Composition of Networked Dispersion of Biocellulose Microfibrils in Water

Skin formulations containing a composition of networked dispersion of biocellulose microfibrils in water were prepared according to the following Table 5, and a degree of attachment of microdust was determined. Artificial sebum was applied to a PMMA plate in an amount of 1.3 mg/cm² and dried thereon, and then the plate was exposed to substitute microdust with a size of 1-5 µm for 5 minutes. Then, the image of microdust attached to the PMMA plate was examined and evaluated.

As a result, when the ratio of microdust attachment in Comparative Example 6 to which no test sample was applied was taken as 100%, it was shown that the microdust attachment ratio was decreased to 94% in the case of Comparative Example 9 and to 70% in the case of Example 5. This suggests that the negative charges contained in the composition of networked dispersion of biocellulose microfibrils repulse the negative charges of microdust to prevent the microdust from being attached to the formulation containing the composition of networked dispersion of biocellulose microfibrils in water.

**[Table 5]**

| | Material | Comp. Ex. 9 | Ex. 5 |
|---|---|---|---|
| 1 | Fucogel | 0.3 | 0.3 |
| 2 | Methylgluceth-20 | 1.00 | 1.00 |
| 3 | Dexpentanol | 0.5 | 0.5 |
| 4 | Polyethylene glycol | 0.4 | 0.4 |
| 5 | Glycerin | 0.5 | 0.5 |
| 6 | Alcohol | 4.0 | 4.0 |
| 7 | Purified water | Up to 100 | Up to 100 |
| 8 | Example 1 | - | 0.05 |
| 9 | Xanthan gum | 0.2 | 0.2 |
| 10 | Carbomer | 0.2 | 0.2 |
| 11 | Preservative | 2 | 2 |
| 12 | Sorbitol | 1.50 | 1.50 |
| 13 | Chelating agent | 0.40 | 0.40 |
| 14 | Anti-inflammatory agent | 2.00 | 2.00 |
| 15 | pH modifier | 0.2 | 0.2 |

**[Table 6]**

| | Comp. Ex. 6 | Comp. Ex. 9 | Ex. 5 |
|---|---|---|---|
| Attachment ratio (%) | 100 | 94 | 70 |

### Test Example 12: Effect of Cleansing Microdust for Formulations Containing Composition of Networked Dispersion of Biocellulose Microfibrils in Water

Essence formulations were prepared according to Table 3 (unit: wt%) and evaluated. When microdust is attached directly to the skin, it infiltrates into pores or wrinkles, thereby making it difficult to clean microdust and cleansing efficiency is decreased. Therefore, a formulation containing a predetermined amount of networked dispersion of biocellulose microfibrils in water and a composition containing the same were applied to the skin, substitute microdust with a size of 1-5 µm was applied thereto, and the skin was cleaned with warm water. Then, the images taken before and after applying substitute microdust and after cleansing were observed to evaluate a microdust cleansing ratio.

As a result, Comparative Example 6 to which no test sample is applied shows a microdust cleansing ratio of 58%. When evaluating the formulations, Comparative Example 9 and Example 9 show an increase in microdust cleansing ratio to 85% and 99%, respectively. This suggests that the composition of networked dispersion of biocellulose microfibrils in water forms a fine network on the skin to inhibit microdust from being attached directly to the skin, and thus microdust can be removed easily during the cleansing.

### Preparation Example 2: Preparation of O/W Emulsion Essence Containing Networked Dispersion of Biocellulose Microfibrils in Water

Essence of oil-in-water (O/W) emulsion formulation containing the networked dispersion of biocellulose microfibrils obtained according to Preparation Example 1 was prepared according to the composition as shown in the following Table 7.

After the networked dispersion of biocellulose microfibrils was charged to purified water and allowed to be wetted therein sufficiently, it was dispersed homogeneously at 1500 rpm for at least 30 minutes by using a disperser. Next, materials 2-5 were added and the mixture was heated to 60°C. Then, the mixture was mixed with materials 7-8 metered and heated to 60°C preliminarily, and then mixing was carried out at 4500 rpm for 10 minutes by using a homomixer. Then, defoaming, filtration and cooling were carried out to finish the formulation.

**[Table 7]**

| No. | Material (wt%) | Example 6 |
|---|---|---|
| 1 | Networked dispersion of biocellulose microfibrils in water | 0.1 |
| 2 | Thickening agent | 0.93 |
| 3 | Xanthan gum | 0.1 |
| 4 | Chelating agent | 0.02 |
| 5 | Preservative | 2 |
| 6 | Purified water | To 100 |
| 7 | Grape seed oil | 0.5 |
| 8 | Emulsifying agent | 0.7 |

### Test Example 13: Keratin Surface Treatment for Composition of Networked Dispersion of Biocellulose Microfibrils in Water

The hair and nail surfaces of a man were treated with the essence of O/W emulsion formulation containing the networked dispersion of biocellulose microfibrils in water according to Example 6, followed by drying. Then, the hair and nail surfaces were observed with scanning electron microscopy (SEM). As shown in FIG. 11 and FIG. 12, it can be seen that the networked dispersion of biocellulose microfibrils in water covers the surfaces while forming a mesh-like network on the surfaces.

### Test Example 14: Comparison of Hair Sectional Area Before and After Treating with Composition of Networked Dispersion of Biocellulose Microfibrils in Water

The hair sample of Test Example 1 was used for the test. The hair surface was measured before and after it was treated with the essence of O/W emulsion formulation containing the networked dispersion of biocellulose microfibrils in water according to Example 6. The results are shown in the following Table 8. The average of the sectional areas of 10 hair samples is about 4012 µm before the treatment with the essence, and was increased to about 4350 µm² by about 8% after the treatment with the essence. Therefore, it can be seen that the networked dispersion of biocellulose microfibrils is coated stably even after the essence formulation was dried on the hair surface, and thus the hair shows an increased thickness.

**[Table 8]**

| No. | Sectional area before treatment (µm²) | Sectional area after treatment (µm²) |
|---|---|---|
| 1 | 4210 | 4406 |
| 2 | 3873 | 4157 |
| 3 | 3294 | 3598 |
| 4 | 3149 | 3456 |
| 5 | 3269 | 3575 |
| 6 | 5323 | 5771 |
| 7 | 5480 | 6085 |
| 8 | 4739 | 5018 |
| 9 | 3541 | 3922 |
| 10 | 3240 | 3509 |
| Average | 4011.8 | 4349.7 |

### Test Example 15: Evaluation of Nail Before and After Treating with Composition of Networked Dispersion of Biocellulose Microfibrils in Water

Two subjects whose nails were damaged by treating them with acetone for removal gel nail once a day for 5 days were allowed to use the essence of O/W emulsion formulation containing the networked dispersion of biocellulose microfibrils in water according to Example 6 on their nails twice a day for 2 weeks. Then, the gloss of each nail was measured by using Skin-glossymeter GL200® (Courage+ Khazaka Electronic GmbH). The results are shown in the following Table 9. When using the essence containing the networked dispersion of biocellulose microfibrils in water, the nail shows significantly increased gloss as compared to the non-treated control. This demonstrates that the networked dispersion of biocellulose microfibrils in water forms a protective net on the surface of nail dried due to the use of an organic solvent, such as acetone, and damaged with the surface cuticle layer separated from the nail, and thus prevents separation of the nail surface keratin layer and assists recovery of the nail, as determined by such increased gloss.

**[Table 9]**

| | Increment of nail gloss as compared to nail before using essence (%) | |
|---|---|---|
| | Control | Test Example |
| Subject 1 | -5.01 | +47.64 |
| Subject 2 | -5.24 | +9.81 |

## Claims

1. A networked dispersion of bacterial cellulose microfibrils in water, which has a number average diameter of 30-60 nm and a maximum diameter of 60-100 nm, and shows a negative charge of -60 mV to -90 mV,
wherein the alcohol groups of bacterial cellulose are substituted with carboxyl groups, and the bacterial cellulose microfibrils are dispersed in an aqueous phase while forming a network, and
wherein at least 0.8 mmol/g of cellulose of the total alcohol groups contained in the bacterial cellulose are substituted with carboxyl groups.

2. The networked dispersion of bacterial cellulose microfibrils in water according to claim 1, which has skin setting property, water absorption-enhancing ability, elasticity-enhancing ability or softness-enhancing ability.

3. The networked dispersion of bacterial cellulose microfibrils in water according to claim 1, which has microdust-interrupting ability.

4. The networked dispersion of bacterial cellulose microfibrils in water according to claim 1, which has keratin structure-protecting ability.

5. A composition comprising the networked dispersion of bacterial cellulose microfibrils in water as defined in any one of claims 1 to 4.

6. The composition according to claim 5, which is a cosmetic composition or sanitary aid composition.

7. The composition according to claim 5, which is a composition for use in enhancing water absorption, enhancing elasticity, interrupting microdust or protecting a keratin structure.

8. The composition according to claim 5, which comprises an aqueous phase.

9. A method for enhancing water absorption or enhancing elasticity by applying the networked dispersion of bacterial cellulose microfibrils in water as defined in any one of claims 1 to 4 or a composition comprising the same to the human body.

10. Non-therapeutic method for interrupting microdust by applying the networked dispersion of bacterial cellulose microfibrils in water as defined in any one of claims 1 to 4 or a composition comprising the same to the human body.

11. Non-therapeutic method for protecting a keratin structure by applying the networked dispersion of bacterial cellulose microfibrils in water as defined in any one of claims 1 to 2 or a composition comprising the same to the human body.

12. The method for protecting a keratin structure according to claim 11, wherein the human body is selected from the group consisting of nail, toenail and body hair.

13. The method for protecting a keratin structure according to claim 12, which is formulated into at least one formulation selected from the group consisting of a pre-shampoo composition, shampoo, rinse, treatment, wax, gel, spray, moose, hair lotion, hair pack, hair essence, hair cream, permanent dyeing agent, temporary dyeing agent, perming agent, nonwoven web and a sheet, when being applied to hair.

14. The method for protecting a keratin structure according to claim 12, which is formulated into at least one formulation selected from the group consisting of a base for trimming or protecting nail and toenail, manicure, topcoat, nourishing agent, reinforcing agent and gel, when being applied to nail or toenail.

## Patentansprüche

1. Vernetzte Dispersion von bakteriellen Cellulose-Mikrofibrillen in Wasser, die einen zahlenmittleren Durchmesser von 30-60 nm und einen maximalen Durchmesser von 60-100 nm aufweist und eine negative Ladung von -60 mV bis -90 mV aufzeigt,
wobei die Alkoholgruppen der bakteriellen Cellulose mit Carboxylgruppen substituiert sind und die bakteriellen Cellulose-Mikrofibrillen in einer wässrigen Phase dispergiert sind, während sie ein Netzwerk bilden, und
wobei mindestens 0,8 mmol/g Cellulose der gesamten Alkoholgruppen, die in der bakteriellen Cellulose enthalten sind, mit Carboxylgruppen substituiert sind.

2. Vernetzte Dispersion von bakteriellen Cellulose-Mikrofibrillen in Wasser nach Anspruch 1, die eine Hautabbindeeigenschaft, eine Wasserabsorptionsverbesserungsfähigkeit, eine Elastizitätsverbesserungsfähigkeit oder eine Weichheitsverbesserungsfähigkeit aufweist.

3. Vernetzte Dispersion von bakteriellen Cellulose-Mikrofibrillen in Wasser nach Anspruch 1, die eine Mikrostaubunterbrechungsfähigkeit aufweist.

4. Vernetzte Dispersion von bakteriellen Cellulose-Mikrofibrillen in Wasser nach Anspruch 1, die eine Keratinstrukturschutzfähigkeit aufweist.

5. Zusammensetzung, die die vernetzte Dispersion von bakteriellen Cellulose-Mikrofibrillen in Wasser nach einem der Ansprüche 1 bis 4 umfasst.

6. Zusammensetzung nach Anspruch 5, die eine kosmetische Zusammensetzung oder eine Sanitärhilfsmittelzusammensetzung ist.

7. Zusammensetzung nach Anspruch 5, die eine Zusammensetzung zur Verwendung bei der Verbesserung der Wasserabsorption, der Verbesserung der Elastizität, der Störung von Mikrostaub oder des Schutzes einer Keratinstruktur ist.

8. Zusammensetzung nach Anspruch 5, die eine wässrige Phase umfasst.

9. Verfahren zur Verbesserung der Wasserabsorption oder zur Verbesserung der Elastizität durch Aufbringen der vernetzten Dispersion von bakteriellen Cellulose-Mikrofibrillen in Wasser nach einem der Ansprüche 1 bis 4 oder einer Zusammensetzung, die diese umfasst, auf den menschlichen Körper.

10. Nicht-therapeutisches Verfahren zur Störung von Mikrostaub durch Aufbringen der vernetzten Dispersion von bakteriellen Cellulose-Mikrofibrillen in Wasser nach einem der Ansprüche 1 bis 4 oder einer Zusammensetzung, die diese umfasst, auf den menschlichen Körper.

11. Nicht-therapeutisches Verfahren zum Schutz einer Keratinstruktur durch Aufbringen der vernetzten Dispersion von bakteriellen Cellulose-Mikrofibrillen in Wasser nach einem der Ansprüche 1 bis 2 oder einer Zusammensetzung, die diese umfasst, auf den menschlichen Körper.

12. Verfahren zum Schutz einer Keratinstruktur nach Anspruch 11, bei der der menschliche Körper aus der Gruppe bestehend aus Nagel, Zehennagel und Körperhaar ausgewählt ist.

13. Verfahren zum Schutz einer Keratinstruktur nach Anspruch 12, das in mindestens eine Formulierung formuliert ist, die aus der Gruppe bestehend aus einer Vorshampoozusammensetzung, einem Shampoo, einer Spülung, einer Behandlung, einem Wachs, einem Gel, einem Spray, einem Moos, einer Haarlotion, einer Haarpackung, einer Haaressenz, einer Haarcreme, einem Permanentfärbemittel, einem temporären Färbemittel, einem Mittel für Dauerwellen, einem Vliesstoff und einer Folie ausgewählt ist, wenn sie auf das Haar aufgebracht wird.

14. Verfahren zum Schutz einer Keratinstruktur nach Anspruch 12, das in mindestens eine Formulierung formuliert ist, die aus der Gruppe bestehend aus einer Basis zum Trimmen oder Schützen von Nagel und Zehennagel, einer Maniküre, einer Deckschicht, einem Pflegemittel, einem Verstärkungsmittel und einem Gel ausgewählt ist, wenn sie auf Nagel oder Zehennagel aufgebracht wird.

## Revendications

1. Dispersion en réseau dans l'eau de microfibrilles d'une cellulose bactérienne ayant un diamètre moyen en nombre de 30-60 nm et un diamètre maximum de 60-100 nm et présentant une charge négative qui va de -60 mV à -90 mV,
où les groupements alcools de la cellulose bactérienne sont substitués par des groupements carboxyles et où les microfibrilles de cellulose bactérienne sont dispersées dans une phase aqueuse tout en formant un réseau, et
où le taux du total des groupements alcools substitués par des groupements carboxyles contenus dans la cellulose bactérienne est d'au moins 0,8 mmol/g de cellulose.

2. Dispersion en réseau dans l'eau de microfibrilles de cellulose bactérienne de la revendication 1, qui possède une propriété de tenue sur la peau, la capacité d'augmenter l'absorption d'eau, la capacité d'augmenter l'élasticité ou la capacité d'augmenter la douceur.

3. Dispersion en réseau dans l'eau de microfibrilles de cellulose bactérienne de la revendication 1, qui possède la capacité de faire barrière aux micropoussières.

4. Dispersion en réseau dans l'eau de microfibrilles de cellulose bactérienne de la revendication 1, qui possède la capacité de protéger une structure kératinisée.

5. Composition comprenant la dispersion en réseau dans l'eau de microfibrilles de cellulose bactérienne telle que définie à l'une quelconque des revendications 1 à 4.

6. Composition selon la revendication 5, qui est une composition cosmétique ou composition de soutien sanitaire.

7. Composition selon la revendication 5, qui est une composition destinée à être utilisée pour augmenter l'absorption d'eau, augmenter l'élasticité, faire barrière aux micropoussières ou protéger une structure kératinisée.

8. Composition selon la revendication 5, qui comprend une phase aqueuse.

9. Méthode permettant d'augmenter l'absorption d'eau ou d'augmenter l'élasticité par application sur le corps humain de la dispersion en réseau dans l'eau de microfibrilles de cellulose bactérienne telle que définie à l'une quelconque des revendications 1 à 4 ou d'une composition la comprenant.

10. Méthode non thérapeutique permettant de faire barrière aux micropoussières par application sur le corps humain de la dispersion en réseau dans l'eau de microfibrilles de cellulose bactérienne telle que définie à l'une quelconque des revendications 1 à 4 ou d'une composition la comprenant.

11. Méthode non thérapeutique permettant de protéger une structure kératinisée par application sur le corps humain de la dispersion en réseau dans l'eau de microfibrilles de cellulose bactérienne telle que définie à l'une quelconque des revendications 1 à 2 ou d'une composition la comprenant.

12. Méthode permettant de protéger une structure kératinisée selon la revendication 11, où le corps humain est sélectionné dans le groupe consistant en l'ongle d'un doigt, l'ongle d'un orteil ou un poil ou cheveu.

13. Méthode permettant de protéger une structure kératinisée selon la revendication 12, qui est formulée sous la forme d'au moins une formulation sélectionnée dans le groupe consistant en une composition pré-shampooing, un shampooing, un après-shampooing, une composition traitante, une cire, un gel, un spray, une mousse, une lotion capillaire, un masque capillaire, une essence capillaire, une crème capillaire, un agent de teinture capillaire permanente, un agent de teinture capillaire temporaire, un agent de permanente, une nappe non tissée et un masque en feuille destiné à un usage capillaire.

14. Méthode permettant de protéger une structure kératinisée selon la revendication 12, qui est formulée sous la forme d'au moins une formulation sélectionnée dans le groupe consistant en une base pour couper ou protéger les ongles des doigts et des orteils, un produit de manucure, un vernis de finition, un agent nourrissant, un agent renforçant et un gel destiné à être appliqué sur l'ongle d'un doigt ou d'un orteil.
